# EUROPEAN PATENT SPECIFICATION

(11) **EP 4 337 159 B1**
(45) Date of publication and mention of the grant of the patent: **05.11.2025**
(21) Application number: 21734536.2
(22) Date of filing: 14.05.2021
(51) Int. Cl.: A61K 8/60, A61K 8/34, A61Q 19/02, A61K 8/67, A61K 8/06, A61K 8/368, A61Q 19/00, A61K 8/37

(54) **COMPOSITION COMPRISING SKIN CARE ACTIVE INGREDIENT AND TWO POLYGLYCERYL FATTY ACID ESTERS**
ZUSAMMENSETZUNG, DIE EINEN HAUTPFLEGEWIRKSTOFF UND ZWEI POLYGLYCERYLFETTSÄUREESTER ENTHÄLT
COMPOSITION COMPRENANT UN INGRÉDIENT ACTIF DE SOIN DE LA PEAU ET DEUX ESTERS D'ACIDES GRAS POLYGLYCÉRIQUES

(43) Date of publication of application: 20.03.2024
(73) Proprietor: L'OREAL, 75008 Paris (FR); Iima, Yusuke, Kawasaki-shi, Kanagawa 213-0012 (JP); Saito, Makoto, Kawasaki-shi, Kanagawa, 213-0012 (JP); Tachon, Romain, Takatsu-ku Kawasaki-shi Kanagawa 213-0012 (JP); Niimi, Rui, Takatsu-ku Kawasaki-shi Kanagawa 213-0012 (JP)
(72) Inventor: IIMA, Yusuke, Kawasaki-shi, Kanagawa 2130012 (JP); SAITO, Makoto, Kawasaki-shi, Kanagawa 2130012 (JP); TACHON, Romain, Kawasaki-shi, Kanagawa 2130012 (JP); NIIMI, Rui, Kawasaki-shi, Kanagawa 2130012 (JP)
(74) Representative: Casalonga
(86) International application number: PCT/JP2021/019485
(87) International publication number: WO 2022/239257

(56) References cited:
- WO-A1-2014/098266
- WO-A1-2015/198922
- WO-A1-2017/074982
- WO-A1-2018/180315
- WO-A1-2019/244908
- WO-A1-2020/110716

## Description

### TECHNICAL FIELD

The present invention relates to a composition, preferably a cosmetic or dermatological composition, which comprises at least one skin care active ingredient and at least two different types of polyglyceryl fatty acid esters.

### BACKGROUND ART

There have been a wide variety of skin care active ingredients used in the field of cosmetics and dermatological compositions.

It is preferable for skin care active ingredients to be absorbed by the skin or penetrated into the skin as much as possible. However, skin absorption or skin penetration of such skin care active ingredients is not easy due to the barrier function of the skin. Thus, one possible approach to enhance the skin penetration of a skin care active ingredient may be to use a skin penetration enhancer with the skin care active ingredient.

For example, WO 2018/097303 uses a combination of a single polyglyceryl fatty acid ester and niacinamide in a nano- or micro-emulsion including a ceramide in order to enhance the skin penetration of the ceramide. See also WO 2015/198922 A1 (OREAL [FR]; IKEDA YUICHI [JP] ET AL.) 30 December 2015 (2015-12-30):

### DISCLOSURE OF INVENTION

There has still been a need for a composition including a skin care active ingredient which can enhance or improve the penetration of the skin care active ingredient into the skin, based on a new skin penetration enhancer.

An objective of the present invention is to provide a composition which can enhance or improve the penetration of a certain class of skin care active ingredients into the skin by using a new skin penetration enhancer.

The above objective of the present invention can be achieved with a composition comprising:
(b) at least one first polyglyceryl fatty acid ester having an HLB value 13.0 or more;
(c) at least one second polyglyceryl fatty acid ester having an HLB value of 10.0 or less, preferably 9.5 or less, and more preferably 9.0 or less;
(d) at least one skin care active ingredient; and
(e) water,
wherein the (d) skin care active ingredient is selected from the group consisting of niacinamide, ascorbyl glucoside, phenylethyl resorcinol, hydroxypropyl tetrahydropyrantriol, 3-O-ethyl ascorbic acid, salicylic acid, and a mixture thereof.

The average HLB of the (b) first polyglyceryl fatty acid ester(s) and the (c) second polyglyceryl fatty acid ester(s) in the composition according to the present invention may range from 12.5 to 13.0.

The (b) first polyglyceryl fatty acid ester may comprise 2 to 4 glycerol units, preferably 3 or 4 glycerol units, and more preferably 4 glycerol units.

The fatty acid moiety of the (b) first polyglyceryl fatty acid ester may comprise 12 or fewer carbon atoms, preferably 11 or fewer carbon atoms, and more preferably 10 or fewer carbon atoms.

The amount of the (b) first polyglyceryl fatty acid ester(s) in the composition according to the present invention may range from 0.01% to 15% by weight, preferably from 0.05% to 10% by weight, and more preferably from 0.1% to 5% by weight, relative to the total weight of the composition.

The (c) second polyglyceryl fatty acid ester may comprise 2 to 4 glycerol units, preferably 2 or 3 glycerol units, and more preferably 2 glycerol units.

The fatty acid moiety of the (c) second polyglyceryl fatty acid ester may comprise 14 or more carbon atoms, preferably 16 or more carbon atoms, and more preferably 18 or more carbon atoms.

The amount of the (c) second polyglyceryl fatty acid ester(s) in the composition according to the present invention may range from 0.01% to 10% by weight, preferably from 0.05% to 5% by weight, and more preferably from 0.1% to 1% by weight, relative to the total weight of the composition.

The weight ratio of the amount of the (b) first polyglyceryl fatty acid ester(s)/the (c) second polyglyceryl fatty acid ester(s) may be from 1 to 5, preferably from 1.5 to 4, and more preferably from 2 to 3.

The (d) skin care active ingredient may have a logP value ranging from -4.5 to 4.5, preferably from - 4.0 to 4.0, and more preferably from -3.5 to 3.5

The (d) skin care active ingredient is selected from the group consisting of niacinamide, ascorbyl glucoside, phenylethyl resorcinol, hydroxypropyl tetrahydropyrantriol, 3-O-ethyl ascorbic acid, salicylic acid, and a mixture thereof.

The amount of the (d) skin care active ingredient(s) in the composition according to the present invention may range from 0.01% to 20% by weight, preferably from 0.05% to 15% by weight, and more preferably from 0.1% to 10% by weight, relative to the total weight of the composition.

The composition according to the present invention may further comprise (a) at least one oil.

The amount of the (a) oil(s) in the composition according to the present invention may range from 0.01% to 20% by weight, preferably from 0.05% to 15% by weight, and more preferably from 0.1% to 10% by weight, relative to the total weight of the composition.

If the composition according to the present invention comprises (a) at least one oil, the composition according to the present invention may be in the form of a nano- or micro-emulsion.

The present invention also relates to a cosmetic process for treating the skin, comprising the step of applying the composition according to the present invention to the skin.

### BEST MODE FOR CARRYING OUT THE INVENTION

After diligent research, the inventors have discovered that it is possible to provide a composition which can enhance or improve the penetration of a certain class of skin care active ingredients into the skin by using a new skin penetration enhancer.

The present invention is as defined in the appended claims and is characterized by the use of a combination of:
at least one first polyglyceryl fatty acid ester having a higher HLB value; and
at least one second polyglyceryl fatty acid ester having a lower HLB value in a composition comprising a skin care active ingredient in order to enhance or improve the penetration of the skin care active ingredient.

The composition according to the present invention may further comprise (a) at least one oil.

The composition according to the present invention can enhance or improve the penetration of the (d) skin care active ingredient into the skin as compared to a case in which the combination of the (b) first polyglyceryl fatty acid ester having an HLB value of 13.0 or more and the (c) second polyglyceryl fatty acid ester having an HLB value of 10.0 or less, preferably 9.5 or less, and more preferably 9.0 or less is not used.

The composition according to the present invention may be transparent or translucent.

It is preferable that the composition according to the present invention be stable such that no or little phase separation is caused even after being stored for a long period of time under elevated temperature.

The composition according to the present invention is environmentally friendly because it uses polyglyceryl fatty acid esters, as surfactants, which can be prepared by raw materials derived from natural resources such as plants.

Further, the composition according to the present invention may be less sticky, and therefore, it can provide a reduced sticky feeling to the touch. The term "sticky" here means a property which provides a tacky feeling to the skin. Therefore, the composition according to the present invention can provide an excellent feeling during use, in particular, feeling of the skin after application of the composition.

Furthermore, the composition according to the present invention may be prepared without a large amount of energy such as that required by a homogenizer. Thus, the composition according to the present invention may be prepared by using a small amount of energy such as gently stirring the ingredients of the composition. Therefore, the composition according to the present invention is environmentally friendly in view of the preparation approach thereof.

Hereinafter, the composition according to the present invention will be explained in a more detailed manner.

### [Oil]

The composition according to the present invention may comprise (a) at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The (a) oil may be a non-polar oil such as a hydrocarbon oil, a silicone oil, or the like; a polar oil such as a plant or animal oil and an ester oil or an ether oil; or a mixture thereof.

The (a) oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula:
   with 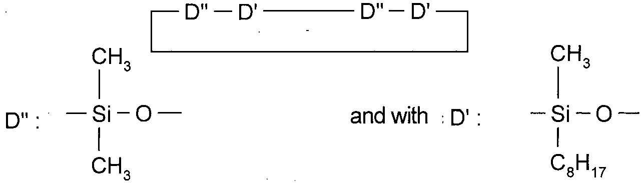 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 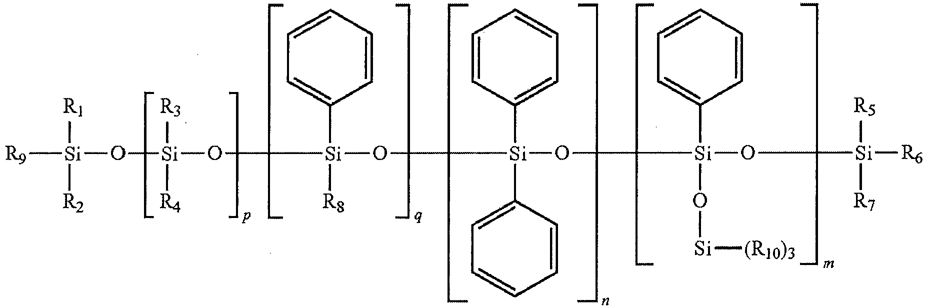 in which
R₁ to R₁₀ independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (R₁to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

The hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from cetyl alcohol, octyldodecanol, hexyldecanol, and mixtures thereof.

It is also preferable that the (a) oil be chosen from oils with a molecular weight below 600 g/mol.

Preferably, the (a) oil has a low molecular weight such as below 600 g/mol, chosen among ester oils with a short hydrocarbon chain or chains (C₁-C₁₂) (e.g., isopropyl lauroyl sarcosinate, isopropyl myristate, isopropyl palmitate, isononyl isononanoate, and ethyl hexyl palmitate), silicone oils (e.g., volatile silicones such as cyclohexasiloxane), hydrocarbon oils (e.g., isododecane, isohexadecane, and squalane), branched and/or unsaturated fatty alcohol (C₁₂-C₃₀) type oils such as octyldodecanol and oleyl alcohol, and ether oils such as dicaprylyl ether.

It is preferable that the (a) oil be chosen from polar oils, and more preferably from ester oils.

The amount of the (a) oil(s) in the composition according to the present invention may be 0.01% by weight or more, preferably 0.05% by weight or more, and more preferably 0.1% by weight or more, relative to the total weight of the composition.

The amount of the (a) oil(s) in the composition according to the present invention may be 20% by weight or less, preferably 15% by weight or less, and more preferably 10% by weight or less, relative to the total weight of the composition.

The amount of the (a) oil(s) in the composition according to the present invention may be from 0.01% to 20% by weight, preferably from 0.05% to 15% by weight, and more preferably from 0.1% to 10% by weight, relative to the total weight of the composition.

### [First Polyglyceryl Fatty Acid Ester]

The composition according to the present invention comprises (b) at least one first polyglyceryl fatty acid ester having an HLB value of 13.0 or more. A single type of (b) first polyglyceryl fatty acid ester may be used, but two or more different types of (b) first polyglyceryl fatty acid ester may be used in combination.

The (b) first polyglyceryl fatty acid ester can function as a surfactant, in particular a nonionic surfactant.

The (b) first polyglyceryl fatty acid ester may have an HLB value 13.5 to 15.0.

The term HLB ("hydrophilic-lipophilic balance") is well known to those skilled in the art, and reflects the ratio between the hydrophilic part and the lipophilic part in the molecule.

If two or more (b) first polyglyceryl fatty acid esters are used, the HLB value is determined by a weighted average of the HLB values of all the (b) first polyglyceryl fatty acid esters.

The (b) first polyglyceryl fatty acid ester may be chosen from mono, di, tri and more esters of saturated or unsaturated fatty acid(s).

It is preferable that the (b) first polyglyceryl fatty acid ester comprise 2 to 4 glycerol units, preferably 3 or 4 glycerol units, and more preferably 4 glycerol units.

The fatty acid for the fatty acid moiety or the fatty acid moiety of the (b) first polyglyceryl fatty acid ester may comprise 12 or fewer carbon atoms, preferably 11 or fewer carbon atoms, and more preferably 10 or fewer carbon atoms. The fatty acid for the fatty acid moiety or the fatty acid moiety of the (b) first polyglyceryl fatty acid ester may comprise 4 or more carbon atoms, preferably 6 or more carbon atoms, and more preferably 8 or more carbon atoms. The fatty acid for the fatty acid moiety or the fatty acid moiety of the (b) first polyglyceryl fatty acid ester may have from 4 to 12 carbon atoms, preferably from 6 to 11 carbon atoms, and more preferably from 8 to 10 carbon atoms.

The fatty acid for the fatty acid moiety of the (b) first polyglyceryl fatty acid ester may be saturated or unsaturated, and may be selected from caprylic acid, capric acid, and lauric acid.

The (b) first polyglyceryl fatty acid ester(s) may be selected from the group consisting of PG3 caprate (HLB: about 14), PG4 caprylate (HLB: 14), PG4 laurate (HLB: about 14), PG4 caprate (HLB: 14), PG5 myristate (HLB: 15.4), PG5 stearate (HLB: 15), PG6 caprylate (HLB: 14.6), PG6 caprate (HLB: 13.1), PG6 laurate (HLB: 14.1), PG10 laurate (HLB: 15.2), PG10 myristate (HLB: 14.9), PG10 stearate (HLB: 14.1), PG10 isostearate (HLB: 13.7), PG10 oleate (HLB: 13.0), PG10 cocoate (HLB: 16), and mixtures thereof.

It is preferable that the (b) first polyglyceryl fatty acid ester(s) be selected from the group consisting of PG3 caprate (HLB: about 14), PG4 caprylate (HLB: 14), PG4 laurate (HLB: about 14), PG4 caprate (HLB: 14), and mixtures thereof.

The amount of the (b) first polyglyceryl fatty acid ester(s) in the composition according to the present invention may be 0.01% by weight or more, preferably 0.05% by weight or more, and more preferably 0.1% by weight or more, relative to the total weight of the composition.

On the other hand, the amount of the (b) first polyglyceryl fatty acid ester(s) in the composition according to the present invention may be 15% by weight or less, preferably 10% by weight or less, and more preferably 5% by weight or less, relative to the total weight of the composition.

The amount of the (b) first polyglyceryl fatty acid ester(s) in the composition according to the present invention may range from 0.01% to 15% by weight, preferably from 0.05% to 10% by weight, and more preferably from 0.1% to 5% by weight, relative to the total weight of the composition.

The weight ratio of the amount of the (b) first polyglyceryl fatty acid ester(s)/the amount of the (a) oil(s), if present, in the composition according to the present invention may range from 0.6 to 1.2, preferably from 0.7 to 1.1, and more preferably from 0.8 to 1.0.

### [Second Polyglyceryl Fatty Acid Ester]

The composition according to the present invention comprises (c) at least one second polyglyceryl fatty acid ester having an HLB value of 10.0 or less, preferably 9.5 or less, and more preferably 9.0 or less. A single type of (c) second polyglyceryl fatty acid ester may be used, but two or more different types of (c) second polyglyceryl fatty acid ester may be used in combination.

The (c) second polyglyceryl fatty acid ester can function as a surfactant, in particular a nonionic surfactant.

The (c) second polyglyceryl fatty acid ester may have an HLB value of 5.0 to 10.0, preferably 6.0 to 9.5, and more preferably 7.0 to 9.0.

If two or more (c) second polyglyceryl fatty acid esters are used, the HLB value is determined by a weighted average of the HLB values of all the (c) second polyglyceryl fatty acid esters.

The (c) second polyglyceryl fatty acid ester may be chosen from mono, di, tri and more esters of saturated or unsaturated fatty acid(s).

It is preferable that the (c) second polyglyceryl fatty acid ester comprise 2 to 4 glycerol units, preferably 2 or 3 glycerol units, and more preferably 2 glycerol units.

The fatty acid for the fatty acid moiety or the fatty acid moiety of the (c) second polyglyceryl fatty acid ester may comprise 14 or more carbon atoms, preferably 16 or more carbon atoms, and more preferably 18 or more carbon atoms. The fatty acid for the fatty acid moiety or the fatty acid moiety of the (c) second polyglyceryl fatty acid ester may comprise 30 or fewer carbon atoms, preferably 24 or fewer carbon atoms, and more preferably 20 or fewer carbon atoms. The fatty acid for the fatty acid moiety or the fatty acid moiety of the (c) second polyglyceryl fatty acid ester may have from 14 to 30, preferably from 16 to 24, and more preferably from 18 to 20 carbon atoms.

The fatty acid for the fatty acid moiety of the (c) second polyglyceryl fatty acid ester may be saturated or unsaturated, and may be selected from myristic acid, stearic acid, isostearic acid, and oleic acid.

The (c) second polyglyceryl fatty acid ester(s) may be selected from the group consisting of PG2 stearate (HLB: 5.0), PG2 distearate (HLB: 4), PG2 isostearate (HLB: 8), PG2 diisostearate (HLB: 3.2), PG2 triisostearate (HLB: 3), PG2 sesquiisostearate (HLB: about 4), PG2 oleate (HLB: 8), PG2 sesquioleate (HLB: 5.3), PG3 distearate (HLB: 5), PG3 diisostearate (HLB: 5), PG3 dicocoate (HLB: 7), PG5 hexastearate (HLB: 4.0), PG5 trioleate (HLB: 7.0), PG10 pentaoleate (HLB: 6.4), PG2 sesquicaprylate (HLB: about 8), PG2 caprate (HLB: 9.5), PG2 laurate (HLB: 8.5), PG2 myristate (HLB: 10), PG2 isopalmitate (HLB: 9), PG4 oleate (HLB: 10), PG4 stearate (HLB: 9), PG4 isostearate (HLB: 8.2), PG6 distearate (HLB: 8), PG10 distearate (HLB: about 9), PG10 tristearate (HLB: 8), PG10 diisostearate (HLB: 10), PG10 triisostearate (HLB: 8), PG10 tricocoate (HLB: 9), and mixtures thereof.

It is preferable that the (c) second polyglyceryl fatty acid ester be selected from the group consisting of PG2 stearate (HLB: 5.0), PG2 distearate (HLB: 4), PG2 isostearate (HLB: 8), PG2 diisostearate (HLB: 3.2), PG2 triisostearate (HLB: 3), PG2 sesquiisostearate (HLB: about 4), PG2 oleate (HLB: 8), PG2 sesquioleate (HLB: 5.3), PG3 distearate (HLB: 5), PG3 diisostearate (HLB: 5), PG3 dicocoate (HLB: 7), PG2 sesquicaprylate (HLB: about 8), PG2 caprate (HLB: 9.5), PG2 laurate (HLB: 8.5), PG2 myristate (HLB: 10), PG2 isopalmitate (HLB: 9), PG4 oleate (HLB: 10), PG4 stearate (HLB: 9), PG4 isostearate (HLB: 8.2), and mixtures thereof.

The amount of the (c) second polyglyceryl fatty acid ester(s) in the composition according to the present invention may be 0.01% by weight or more, preferably 0.05% by weight or more, and more preferably 0.1% by weight or more, relative to the total weight of the composition.

On the other hand, the amount of the (c) second polyglyceryl fatty acid ester(s) in the composition according to the present invention may be 10% by weight or less, preferably 5% by weight or less, and more preferably 1% by weight or less, relative to the total weight of the composition.

The amount of the (c) second polyglyceryl fatty acid ester(s) in the composition according to the present invention may range from 0.01% to 10% by weight, preferably from 0.05% to 5% by weight, more preferably from 0.1% to 1% by weight, relative to the total weight of the composition.

The weight ratio of the amount of the (c) second polyglyceryl fatty acid ester(s)/the amount of the (a) oil(s), if present, in the composition according to the present invention may range from 0.1 to 0.9, preferably from 0.2 to 0.7, and more preferably from 0.3 to 0.5.

### [Amount of Polyglyceryl Fatty Acid Esters]

According to the present invention, the weight ratio of (the total amounts of the (b) first polyglyceryl fatty acid ester(s) and the (c) second polyglyceryl fatty acid ester(s))/the amount of the (a) oil(s), if present, may be 3.0 or less, preferably 2.0 or less, and more preferably 1.5 or less.

Conventionally, the weight ratio of the amount(s) of glyceryl fatty acid ester(s)/the oil(s), if present, is much higher, such as 6.0.

Therefore, the composition according to the present invention can reduce or limit the total amounts of the polyglyceryl fatty acid esters.

Since the present invention can reduce the total amounts of the polyglyceryl fatty acid esters, the composition according to the present invention may provide no sticky feeling or may provide a further reduced sticky feeling to the touch.

It is preferable that the weight ratio of (the total amounts of the (b) first polyglyceryl fatty acid ester(s) and the (c) second polyglyceryl fatty acid ester(s))/the amount of the (a) oil(s), if present, be more than 0.1, preferably more than 0.2, and more preferably more than 0.3.

On the other hand, according to the present invention, the weight ratio of the amount of the (b) first polyglyceryl fatty acid ester(s)/the (c) second polyglyceryl fatty acid ester(s) may be 1 or more, preferably 1.5 or more, and more preferably 2 or more; may be 5 or less, preferably 4 or less, and more preferably 3 or less; and may be from 1 to 5, preferably from 1.5 to 4, and more preferably from 2 to 3.

### [Average HLB of Polyglyceryl Fatty Acid Esters]

The average HLB value of the (b) first polyglyceryl fatty acid ester(s) and the (c) second polyglyceryl fatty acid ester(s) can be calculated as a weighted average of all the first and second polyglyceryl fatty acid esters.

The average HLB of the (b) first polyglyceryl fatty acid ester(s) and the (c) second polyglyceryl fatty acid ester(s) in the composition according to the present invention may be 12.5 or more.

The average HLB of the (b) first polyglyceryl fatty acid ester(s) and the (c) second polyglyceryl fatty acid ester(s) in the composition according to the present invention may be 14.0 or less, preferably 13.5 or less, and more preferably 13.0 or less.

Thus, in one embodiment of the present invention, the average HLB of the (b) first polyglyceryl fatty acid ester(s) and the (c) second polyglyceryl fatty acid ester(s) in the composition according to the present invention may range from 12.5 to 13.0.

### [Skin Care Active Ingredient]

The composition according to the present invention comprises (d) at least a skin care active ingredient, selected from the groupconsisting of niacinamide, ascorbyl glucoside, phenylethyl resorcinol, hydroxypropyl tetrahydropyrantriol, 3-O-ethyl ascorbic acid, salicylic acid, and a mixture thereof.

It is preferable that the (d) skin care active ingredient have a logP value ranging from -4.5 to 4.5, preferably from -4.0 to 4.0, and more preferably from -3.5 to 3.5.

A log P value is a value for the base-ten logarithm of the apparent octan-1-ol/water partition coefficient. The log p values are known and are determined by a standard test which determines the concentration of the (c) compound in octan-1-ol and water. The log P may be calculated according to the method described in the article by Meylan and Howard: Atom/Fragment contribution method for estimating octanol-water partition coefficients, J. Pharm. Sci., 84: 83-92, 1995. This value may also be calculated using numerous commercially available software packages, which determine the log P as a function of the structure of a molecule. By way of example, mention may be made of the Epiwin software from the United States Environmental Agency.

The values may especially be calculated using the ACD (Advanced Chemistry Development) Solaris software V4.67; they may also be obtained from Exploring QSAR: hydrophobic, electronic and steric constants (ACS professional reference book, 1995). There is also an Internet site which provides estimated values (address: http://esc.syrres.com/interkow/kowdemo.htm).

It is preferable that the (d) skin care active ingredient be a skin care cosmetic active ingredient, and more preferably a skin whitening ingredient or a skin anti-aging ingredient such as a skin anti-wrinkle ingredient.

As the (d) skin care active ingredient, mention may be made of niacinamide.

The amount of the (d) skin care active ingredient in the composition according to the present invention may be 0.01% by weight or more, preferably 0.05% by weight or more, and more preferably 0.1% by weight or more, relative to the total weight of the composition.

On the other hand, the amount of the (d) skin care active ingredient in the composition according to the present invention may be 20% by weight or less, preferably 15% by weight or less, and more preferably 10% by weight or less, relative to the total weight of the composition.

The amount of (d) skin care active ingredient in the composition according to the present invention may range from 0.01% to 20% by weight, preferably from 0.05% to 15% by weight, more preferably from 0.1% to 10% by weight, relative to the total weight of the composition.

### [Water]

The composition according to the present invention comprises (e) water.

The amount of the (e) water in the composition according to the present invention may be 60% by weight or more, preferably 70% by weight or more, and more preferably 80% by weight or more, relative to the total weight of the composition.

On the other hand, the amount of the (e) water in the composition according to the present invention may be 95% by weight or less, preferably 90% by weight or less, and more preferably 85% by weight or less, relative to the total weight of the composition.

The amount of (e) water in the composition according to the present invention may range from 60% to 95% by weight, preferably from 70% to 90% by weight, more preferably from 80% to 85% by weight, relative to the total weight of the composition.

### [Polyol]

The composition according to the present invention may further comprise at least one polyol. A single type of polyol may be used, but two or more different types of polyol may be used in combination.

The term "polyol" here means an alcohol having two or more hydroxy groups, and does not encompass a saccharide or a derivative thereof. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acyl group or a carbonyl group.

The polyol may be a C₂-C₁₂ polyol, preferably a C₂-C₉ polyol, comprising at least 2 hydroxy groups, and preferably 2 to 5 hydroxy groups.

The polyol may be a natural or synthetic polyol. The polyol may have a linear, branched or cyclic molecular structure.

The polyol may be selected from glycerins and derivatives thereof, and glycols and derivatives thereof. The polyol may be selected from the group consisting of glycerin, diglycerin, polyglycerin, ethyleneglycol, diethyleneglycol, propyleneglycol, dipropyleneglycol, butyleneglycol, pentyleneglycol, hexyleneglycol, 1,3-propanediol, 1,5-pentanediol, polyethyleneglycol (5 to 50 ethyleneoxide groups), and sugars such as sorbitol.

The amount of the polyol(s) in the composition according to the present invention may be 0.01% by weight or more, preferably 0.05% by weight or more, and more preferably 0.1% by weight or more, relative to the total weight of the composition.

On the other hand, the amount of the polyol(s) in the composition according to the present invention may be 25% by weight or less, preferably 20% by weight or less, and more preferably 15% by weight or less, relative to the total weight of the composition.

Thus, the polyol(s) may be present in the composition according to the present invention in an amount ranging from 0.01% to 25% by weight, and preferably from 0.05% to 20% by weight, such as from 0.1% to 15% by weight, relative to the total weight of the composition.

### [Other Ingredients]

The composition according to the present invention may contain one or more monoalcohols which are in the form of a liquid at room temperature (25°C), such as for example linear or branched monoalcohols comprising from 1 to 6 carbon atoms, such as ethanol, propanol, butanol, isopropanol, isobutanol, pentanol, and hexanol.

The amount of the monoalcohol(s) in the composition according to the present invention may be 0.01% by weight or more, preferably 0.1% by weight or more, and more preferably 1% by weight or more, relative to the total weight of the composition.

On the other hand, the amount of the monoalcohol(s) in the composition according to the present invention may be 15% by weight or less, preferably 10% by weight or less, and more preferably 5% by weight or less, relative to the total weight of the composition.

Thus, the amount of the monoalcohol(s) in the composition according to the present invention may range from 0.01% to 15% by weight, preferably from 0.1% to 10% by weight, and more preferably from 1% to 5% by weight, relative to the total weight of the composition.

The composition according to the present invention may also include various adjuvants conventionally used in cosmetic and dermatological compositions, such as thickeners, anionic, non-ionic, cationic, and amphoteric or zwitterionic polymers, anionic, non-ionic, cationic, and amphoteric surfactants, antioxidants, coloring agents, chelating agents, sequestering agents, fragrances, dispersing agents, conditioning agents, film-forming agents, preservatives, co-preservatives, and mixtures thereof, except for the ingredients as explained above.

In one embodiment, the composition according to the present invention may be free from a polyglyceryl fatty acid ester comprising 5 or more glycerol units such as PG5 laurate. The term "free from" here means that the composition according to the present invention may contain a limited amount of a polyglyceryl fatty acid ester comprising 5 or more glycerol units. However, it is preferable that the amount of the polyglyceryl fatty acid ester comprising 5 or more glycerol units be limited such that it is less than 1% by weight, more preferably less than 0.1% by weight, and even more preferably less than 0.01% by weight, relative to the total weight of the composition. It is most preferable that the composition according to the present invention comprise no polyglyceryl fatty acid ester comprising 5 or more glycerol units.

In another embodiment, the composition according to the present invention may be free from a polyoxyethylene-based nonionic surfactant. The term "free from" here means that the composition according to the present invention may contain a limited amount of a polyoxyethylene-based nonionic surfactant. However, it is preferable that the amount of the polyoxyethylene-based nonionic surfactant be limited such that it is less than 1% by weight, more preferably less than 0.1% by weight, and even more preferably less than 0.01% by weight, relative to the total weight of the composition. It is most preferable that the composition according to the present invention comprises no polyoxyethylene-based nonionic surfactant.

### (Preparation)

The composition according to the present invention can be prepared by mixing the essential ingredient(s) as explained above, and optional ingredient(s), if necessary, as explained above.

The method and means to mix the above essential and optional ingredients are not limited. Any conventional method and means can be used to mix the above essential and optional ingredients to prepare the composition according to the present invention.

The composition according to the present invention may be prepared without a large amount of energy such as that required by a homogenizer. Thus, the composition according to the present invention may be prepared by using a small amount of energy such as gently stirring the ingredients of the composition. Therefore, the composition according to the present invention is environmentally friendly in view of the preparation approach thereof.

### [Form]

The form of the composition according to the present invention is not limited.

The composition according to the present invention may be in the form of a nano- or micro-emulsion, if the composition according to the present invention comprises (a) at least one oil.

The "micro-emulsion" may be defined in two ways, namely, in a broad sense and in a narrow sense. That is to say, there is the one case ("micro-emulsion in the narrow sense") in which the micro-emulsion refers to a thermodynamically stable isotropic single liquid phase containing a ternary system having three ingredients of an oily component, an aqueous component and a surfactant, and there is the second case ("micro-emulsion in the broad sense") in which among thermodynamically unstable typical emulsion systems the micro-emulsion additionally includes those such emulsions presenting transparent or translucent appearances due to their smaller particle sizes (Satoshi Tomomasa, et al., Oil Chemistry, Vol. 37, No. 11 (1988), pp. 48-53). The "micro-emulsion" as used herein refers to a "micro-emulsion in the narrow sense", i.e., a thermodynamically stable isotropic single liquid phase.

The micro-emulsion refers to either one state of an O/W (oil-in-water) type microemulsion in which oil is solubilized by micelles, a W/O (water-in-oil) type microemulsion in which water is solubilized by reverse micelles, or a bicontinuous microemulsion in which the number of associations of surfactant molecules are rendered infinite so that both the aqueous phase and oil phase have a continuous structure.

The micro-emulsion may have a dispersed phase with a particle size of 100 nm or less, preferably 50 nm or less, and more preferably 20 nm or less, measured by laser granulometry.

The "nano-emulsion" here means an emulsion characterized by a dispersed phase with a size of less than 350 nm, the dispersed phase being stabilized by a crown of the (b) and (c) nonionic surfactants that may optionally form a liquid crystal phase of lamellar type, at the dispersed phase/continuous phase interface. In the absence of specific opacifiers, the transparency of the nano-emulsions arises from the small size of the dispersed phase, this small size being obtained by virtue of the use of mechanical energy.

Nanoemulsions can be distinguished from microemulsions by their structure. Specifically, micro-emulsions are thermodynamically stable dispersions formed from, for example, micelles which are formed by the ingredients (b) and (c) and swollen with the ingredient (a). Furthermore, microemulsions do not require substantial mechanical energy in order to be prepared.

The nano-emulsion may have a dispersed phase with a particle size of 300 nm or less, preferably 200 nm or less, and more preferably 100 nm or less, measured by laser granulometry.

It is preferable that the composition according to the present invention be in the form of an O/W emulsion which comprises oil phases dispersed in a continuous aqueous phase. The dispersed oil phases can be oil droplets in the aqueous phase.

The O/W architecture or structure, which consists of oil phases dispersed in an aqueous phase, has an external aqueous phase, and therefore if the composition according to the present invention has the O/W architecture or structure, it can provide a pleasant feeling during use because of the feeling of immediate freshness that the aqueous phase can provide.

It is even more preferable that the particle size of the (a) oil be 35 nm or less, preferably 30 nm or less, and more preferably 25 nm or less, if the composition according to the present invention is in the form of an O/W emulsion. The particle size can be measured by a dynamic light scattering method. The particle size measurement can be performed by, for example, the Particle Size Analyzer ELSZ-2000 series, marketed by Otsuka Electronics Co., Ltd.

The particle size can be a volume-average particle diameter or a number-average particle diameter, preferably a volume-average particle diameter.

The composition according to the present invention can be transparent.

The transparency may be measured by measuring the turbidity (for example, turbidity can be measured with a 2100Q (marketed by Hach Company) having a round cell (25 mm in diameter and 60 mm height) and a tungsten filament lamp which can emit visible light (between 400 and 800 nm, preferably from 400 to 500 nm). The measurement can be performed on the undiluted composition. The blank may be determined with distilled water.

The composition according to the present invention has a turbidity of 150 NTU or less, preferably 130 NTU or less and more preferably 110 NTU or less.

### [Process and Use]

It is preferable that the composition according to the present invention be a cosmetic or dermatological composition, preferably a cosmetic composition, and more preferably a cosmetic composition for skin.

The composition according to the present invention can be used for a non-therapeutic process, such as a cosmetic process, for treating skin, by being applied to the skin.

Thus, the present invention also relates to a cosmetic process for treating skin, comprising the step of applying the composition according to the present invention to the skin.

The present invention may also relate to a use of the composition according to the present invention as a cosmetic product or in a cosmetic product such as skin care products.

In other words, the composition according to the present invention can be used, as it is, as a cosmetic product. Alternatively, the composition according to the present invention can be used as an element of a cosmetic product. For example the composition according to the present invention can be added to or combined with any other elements to form a cosmetic product.

The skin care product may be a lotion, a cream, a serum, a sun screening agent, and the like.

Another aspect of the present invention also relates to a process for preparing a composition, comprising a step of mixing the ingredients b-e as defined in claim 1.

It is preferable that the mixing step be performed by a so-called low energy process without a special mechanical stirrer, such as a homogenizer which uses a large amount of energy. The low energy process can be performed by simply gently stirring the ingredients (b) to (e).

The above composition in the use and process according to the present invention may further comprise (a) at least one oil. In this case, it is preferable that the above composition in the use and process according to the present invention be in the form of an O/W nano- or micro-emulsion.

Another aspect of the present invention relates to a use of a composition as defined in appended claim 1.

The above explanations regarding the ingredients (b) to (e), as well as the optional ingredients including the ingredient (a), for the composition according to the present invention can apply to those for the uses and processes according to the present invention. The explanations regarding the preparation and forms of the composition according to the present invention can also apply to those of the composition recited in the above uses and processes.

### EXAMPLES

The present invention will be described in more detail by way of examples which however should not be construed as limiting the scope of the present invention.

### [Examples 1-12 and Comparative Examples 1-18]

The following compositions according to Examples 1-12 and Comparative Examples 1-18 shown in Tables 1-6, were prepared by mixing the ingredients shown in Tables 1-6 as follows. The numerical values for the amounts of the ingredients shown in Tables 1-6 are all based on "% by weight" as active materials.

**Table 1**

| | Ingredients | Ex. 1 | Ex. 2 | Comp Ex. 1 | Comp Ex. 2 | Comp Ex. 3 |
|---|---|---|---|---|---|---|
| (d) | ASCORBYL GLUCOSIDE (logP = -3.4) | 2 | 2 | 2 | 2 | 2 |
| (a) | ISOPROPYL MYRISTATE | 1 | - | 1 | 1 | - |
| | ISOPROPYL LAUROYL SARCOSINATE | - | - | - | - | 0.5 |
| | ETHYLHEXYL PALMITATE | - | - | - | - | 0.5 |
| (b) | POLYGLYCERYL-4 CAPRATE | 1.05 | 1.05 | 1.5 | - | - |
| (c) | POLYGLYCERYL-2 OLEATE | 0.45 | 0.45 | - | 1.5 | - |
| S | POLYGLYCERYL-5 LAURATE | - | - | - | - | 1 |
| | PPG-6-DECYLTETRADECETH-30 | - | - | - | - | 1 |
| | SODIUM LAUROYL GLUTAMATE | 0.05 | 0.05 | 0.05 | 0.05 | - |
| | SODIUM METHYL STEAROYL TAURATE | - | - | - | - | 0.2 |
| | METHYL GLUCETH-10 | - | - | - | - | 3 |
| | BIS-PEG-18 METHYL ETHER DIMETHYL SILANE | - | - | - | - | 3.5 |
| P | BUTYLENE GLYCOL | - | - | - | - | 5 |
| | GLYCERIN | 10 | 10 | 10 | 10 | 5 |
| | PENTYLENE GLYCOL | 3 | 3 | 3 | 3 | - |
| | PROPYLENE GLYCOL | 3 | 3 | 3 | 3 | - |
| | ALCOHOL DENAT. | 2 | 2 | 2 | 2 | - |
| | CAPRYLYL GLYCOL | 0.3 | 0.3 | 0.3 | 0.3 | - |
| | PHENOXYETHANOL | - | - | - | - | 0.25 |
| | CHLORPHENESIN | - | - | - | - | 0.27 |
| | DISODIUM EDTA | - | - | - | - | 0.1 |
| | XANTHAN GUM | 0.3 | 0.3 | 0.3 | 0.3 | 0.15 |
| | CITRIC ACID | 0.1 | 0.1 | 0.1 | 0.1 | - |
| | ARGININE | 1.50 | 1.50 | 1.50 | 1.50 | - |
| | POTASSIUM HYDROXIDE | - | - | - | - | 0.1 |
| | WATER | qsp 100 | qsp 100 | qsp 100 | qsp 100 | qsp 100 |
| Stability | | Good | Good | Poor | Poor | Good |
| Penetration Amount of Active Ingredient (%) | | 102-+5 | 106±7 | NA | NA | 85±5 |

**Table 2**

| | Ingredients | Ex. 3 | Ex. 4 | Comp Ex. 4 | Comp Ex. 5 | Comp Ex. 6 |
|---|---|---|---|---|---|---|
| (d) | HYDROXYPROPYLTETRAHYDROPYRANTRIOL (logP = -1.5) | 9 | 9 | 9 | 9 | 9 |
| (a) | ISOPROPYL MYRISTATE | 1 | - | 1 | 1 | - |
| | ISOPROPYL LAUROYL SARCOSINATE | - | - | - | - | 0.5 |
| | ETHYLHEXYL PALMITATE | - | - | - | - | 0.5 |
| (b) | POLYGLYCERYL-4 CAPRATE | 1.05 | 1.05 | 1.5 | - | - |
| (c) | POLYGLYCERYL-2 OLEATE | 0.45 | 0.45 | - | 1.5 | - |
| S | POLYGLYCERYL-5 LAURATE | - | - | - | - | 1 |
| | PPG-6-DECYLTETRADECETH-30 | - | - | - | - | 1 |
| | SODIUM LAUROYL GLUTAMATE | 0.05 | 0.05 | 0.05 | 0.05 | - |
| | SODIUM METHYL STEAROYLTAURATE | - | - | - | - | 0.2 |
| | METHYL GLUCETH-10 | - | - | - | - | 3 |
| | BIS-PEG-18 METHYL ETHER DIMETHYL SILANE | - | - | - | - | 3.5 |
| P | BUTYLENE GLYCOL | - | - | - | - | 5 |
| | GLYCERIN | 10 | 10 | 10 | 10 | 5 |
| | PENTYLENE GLYCOL | 3 | 3 | 3 | 3 | - |
| | PROPYLENE GLYCOL | 3 | 3 | 3 | 3 | - |
| | ALCOHOL DENAT. | 2 | 2 | 2 | 2 | - |
| | CAPRYLYL GLYCOL | 0.3 | 0.3 | 0.3 | 0.3 | - |
| | PHENOXYETHANOL | - | - | - | - | 0.25 |
| | CHLORPHENESIN | - | - | - | - | 0.27 |
| | DISODIUM EDTA | - | - | - | - | 0.1 |
| | XANTHAN GUM | 0.3 | 0.3 | 0.3 | 0.3 | 0.15 |
| | CITRIC ACID | 0.1 | 0.1 | 0.1 | 0.1 | - |
| | ARGININE | 0.41 | 0.41 | 0.41 | 0.41 | - |
| | POTASSIUM HYDROXIDE | - | - | - | - | 0.045 |
| | WATER | qsp 100 | qsp 100 | qsp 100 | qsp 100 | qsp 100 |
| Stability | | Good | Good | Poor | Poor | Good |
| Penetration Amount of Active Ingredient (%) | | 16±5 | 15±5 | NA | NA | 0.19±0.1 |

**Table 3**

| | Ingredients | Ex. 5 | Ex. 6 | Comp Ex. 7 | Comp Ex. 8 | Comp Ex. 9 |
|---|---|---|---|---|---|---|
| (d) | 3-O-ETHYL ASCORBIC ACID (LogP = -1.3) | 1 | 1 | 1 | 1 | 1 |
| (a) | ISOPROPYL MYRISTATE | 1 | - | 1 | 1 | - |
| | ISOPROPYL LAUROYL SARCOSINATE | - | - | - | - | 0.5 |
| | ETHYLHEXYL PALMITATE | - | - | - | - | 0.5 |
| (b) | POLYGLYCERYL4 CAPRATE | 1.05 | 1.05 | 1.5 | - | - |
| (c) | POLYGLYCERYL-2 OLEATE | 0.45 | 0.45 | - | 1.5 | - |
| S | POLYGLYCERYL-5 LAURATE | - | - | - | - | 1 |
| | PPG-6-DECYLTETRADECETH-30 | - | - | - | - | 1 |
| | SODIUM LAUROYL GLUTAMATE | 0.05 | 0.05 | 0.05 | 0.05 | - |
| | SODIUM METHYL STEAROYL TAURATE | - | - | - | - | 0.2 |
| | METHYLGLUCETH-10 | - | - | - | - | 3 |
| | BIS-PEG-18 METHYL ETHER DIMETHYL SILANE | - | - | - | - | 3.5 |
| P | BUTYLENE GLYCOL | - | - | - | - | 5 |
| | GLYCERIN | 10 | 10 | 10 | 10 | 5 |
| | PENTYLENE GLYCOL | 3 | 3 | 3 | 3 | - |
| | PROPYLENE GLYCOL | 3 | 3 | 3 | 3 | - |
| | ALCOHOL DENAT. | 2 | 2 | 2 | 2 | - |
| | CAPRYLYL GLYCOL | 0.4 | 0.4 | 0.4 | 0.4 | - |
| | PHENOXYETHANOL | - | - | - | - | 0.25 |
| | CHLORPHENESIN | - | - | - | - | 0.27 |
| | DISODIUM EDTA | - | - | - | - | 0.1 |
| | XANTHAN GUM | 0.3 | 0.3 | 0.3 | 0.3 | 0.15 |
| | CITRIC ACID | 0.1 | 0.1 | 0.1 | 0.1 | - |
| | ARGININE | 0.23 | 0.23 | 0.23 | 0.23 | - |
| | POTASSIUM HYDROXIDE | - | - | - | - | - |
| | WATER | qsp 100 | qsp 100 | qsp 100 | qsp 100 | qsp 100 |
| Stability | | Good | Good | Poor | Poor | Good |
| Penetration Amount of Active Ingredient (%) | | 109±10 | 105±10 | NA | NA | 59±5 |

**Table 4**

| | Ingredients | Ex. 7 | Ex. 8 | Comp Ex. 10 | Comp Ex. 11 | Comp Ex. 12 |
|---|---|---|---|---|---|---|
| (d) | NIACINAMIDE (LogP = -0.37) | 4 | 4 | 4 | 4 | 4 |
| (a) | ISOPROPYL MYRISTATE | 1 | - | 1 | 1 | - |
| | ISOPROPYL LAUROYL SARCOSINATE | - | - | - | - | 0.5 |
| | ETHYLHEXYLPALMITATE | - | - | - | - | 0.5 |
| (b) | POLYGLYCERYL-4 CAPRATE | 1.05 | 1.05 | 1.5 | - | - |
| (c) | POLYGLYCERYL-2 OLEATE | 0.45 | 0.45 | - | 1.5 | - |
| S | POLYGLYCERYL-5 LAURATE | - | - | - | - | 1 |
| | PPG-6-DECYLTETRADECETH-30 | - | - | - | - | 1 |
| | SODIUM LAUROYL GLUTAMATE | 0.05 | 0.05 | 0.05 | 0.05 | - |
| | SODIUM METHYL STEAROYL TAURATE | - | - | - | - | 0.2 |
| | METHYL GLUCETH-10 | - | - | - | - | 3 |
| | BIS-PEG-18 METHYL ETHER DIMETHYL SILANE | - | - | - | - | 3.5 |
| P | BUTYLENE GLYCOL | - | - | - | - | 5 |
| | GLYCERIN | 10 | 10 | 10 | 10 | 5 |
| | PENTYLENE GLYCOL | 3 | 3 | 3 | 3 | - |
| | PROPYLENE GLYCOL | 3 | 3 | 3 | 3 | - |
| | ALCOHOL DENAT. | 2 | 2 | 2 | 2 | - |
| | CAPRYLYL GLYCOL | 0.3 | 0.3 | 0.3 | 0.3 | - |
| | PHENOXYETHANOL | - | - | - | - | 0.25 |
| | CHLORPHENESIN | - | - | - | - | 0.27 |
| | DISODIUM EDTA | - | - | - | - | 0.1 |
| | XANTHAN GUM | 0.3 | 0.3 | 0.3 | 0.3 | 0.15 |
| | CITRIC ACID | 0.34 | 0.34 | 0.34 | 0.34 | 0.17 |
| | ARGININE | 0.81 | 0.81 | 0.81 | 0.81 | - |
| | POTASSIUM HYDROXIDE | - | - | - | - | 0.18 |
| | WATER | qsp 100 | qsp 100 | qsp 100 | qsp 100 | qsp 100 |
| Stability | | Good | Good | Poor | Poor | Good |
| Penetration Amount of Active Ingredient (%) | | 108±10 | 110±10 | NA | NA | 25±5 |

**Table 5**

| | Ingredients | Ex. 9 | Ex. 10 | Comp Ex. 13 | Comp Ex. 14 | Comp Ex. 15 |
|---|---|---|---|---|---|---|
| (d) | SALICYLIC ACID (LogP = 2.3) | 0.2 | 0.2 | 0.2 | 0.2 | 0.2 |
| (a | ISOPROPYL MYRISTATE | 1 | 1 | 1 | - | - |
| | ISOPROPYL LAUROYL SARCOSINATE | - | - | - | - | 0.5 |
| | ETHYLHEXYL PALMITATE | - | - | - | - | 0.5 |
| (b) | POLYGLYCERYL-4 CAPRATE | 1.05 | - | 1.5 | 1.05 | - |
| (c) | POLYGLYCERYL-2 OLEATE | 0.45 | 1.5 | - | 0.45 | - |
| S | POLYGLYCERYL-5 LAURATE | - | - | - | - | 1 |
| | PPG-6-DECYLTETRADECETH-30 | - | - | - | - | 1 |
| | SODIUM LAUROYL GLUTAMATE | 0.05 | 0.05 | 0.05 | 0.05 | - |
| | SODIUM METHYL STEAROYL TAURATE | - | - | - | - | 0.2 |
| | METHYL GLUCETH-10 | - | - | - | - | 3 |
| | BIS-PEG-18 METHYL ETHER DIMETHYL SILANE | - | - | - | - | 3.5 |
| p | BUTYLENE GLYCOL | - | - | - | - | 5 |
| | GLYCERIN | 10 | 10 | 10 | 10 | 5 |
| | PENTYLENE GLYCOL | 3 | 3 | 3 | 3 | - |
| | PROPYLENE GLYCOL | 3 | 3 | 3 | 3 | - |
| | ALCOHOL DENAT. | 2 | 2 | 2 | 2 | - |
| | CAPRYLYL GLYCOL | 0.4 | 0.4 | 0.4 | 0.4 | - |
| | PHENOXYETHANOL | - | - | - | - | 0.25 |
| | CHLORPHENESIN | - | - | - | - | 0.27 |
| | DISODIUM EDTA | - | - | - | - | 0.1 |
| | XANTHAN GUM | 0.2 | 0.2 | 0.2 | 0.2 | 0.15 |
| | CITRIC ACID | 0.1 | 0.1 | 0.1 | 0.1 | - |
| | ARGININE | 0.53 | 0.53 | 0.53 | 0.53 | - |
| | POTASSIUM HYDROXIDE | - | - | - | - | 0.045 |
| | WATER | qsp 100 | qsp 100 | qsp 100 | qsp 100 | qsp 100 |
| Stability | | Good | NA | Poor | Good | Good |
| Penetration Amount of Active Ingredient (%) | | 94±9 | NA | NA | 99±12 | 72±5 |

**Table 6**

| | Ingredients | Ex. 11 | Ex. 12 | Comp Ex. 16 | Comp Ex. 17 | Comp Ex. 18 |
|---|---|---|---|---|---|---|
| (d) | PHENYLETHYL RESORCINOL(logP = 3.07) | 0.3 | 0.3 | 0.3 | 0.3 | 0.3 |
| (a) | ISOPROPYL MYRISTATE | 1 | - | 1 | 1 | - |
| | ISOPROPYL LAUROYL SARCOSINATE | - | - | - | - | 0.5 |
| | ETHYLHEXYL PALMITATE | - | - | - | - | 0.5 |
| (b) | POLYGLYCERYL-4 CAPRATE | 1.05 | 1.05 | 1.5 | - | - |
| (c) | POLYGLYCERYL-2 OLEATE | 0.45 | 0.45 | - | 1.5 | - |
| S | POLYGLYCERYL-5 LAURATE | - | - | - | - | 1 |
| | PPG-6-DECYLTETRADECETH-30 | - | - | - | - | 1 |
| | SODIUM LAUROYL GLUTAMATE | 0.05 | 0.05 | 0.05 | 0.05 | - |
| | SODIUM METHYL STEAROYL TAURATE | - | - | - | - | 0.2 |
| | METHYL GLUCETH-10 | - | - | - | - | 3 |
| | BIS-PEG-18 METHYL ETHER DIMETHYL SILANE | - | - | - | - | 3.5 |
| P | BUTYLENE GLYCOL | - | - | - | - | 5 |
| | GLYCERIN | 10 | 10 | 10 | 10 | 5 |
| | PENTYLENE GLYCOL | 3 | 3 | 3 | 3 | - |
| | PROPYLENE GLYCOL | 3 | 3 | 3 | 3 | - |
| | ALCOHOL DENAT. | 2 | 2 | 2 | 2 | 2 |
| | CAPRYLYL GLYCOL | 0.4 | 0.4 | 0.4 | 0.4 | - |
| | PHENOXYETHANOL | - | - | - | - | 0.25 |
| | CHLORPHENESIN | - | - | - | - | 0.27 |
| | DISODIUM EDTA | - | - | - | - | 0.1 |
| | XANTHAN GUM | 0.2 | 0.2 | 0.2 | 0.2 | 0.15 |
| | CITRIC ACID | 0.1 | 0.1 | 0.1 | 0.1 | - |
| | ARGININE | 0.5 | 0.5 | 0.5 | 0.5 | - |
| | POTASSIUM HYDROXIDE | - | - | - | - | 0.045 |
| | WATER | qsp 100 | qsp 100 | qsp 100 | qsp 100 | qsp 100 |
| Stability | | Good | Good | Poor | Poor | Good |
| Penetration Amount of Active Ingredient (%) | | 14±5 | 15+7 | NA | NA | 8±3 |

| | | | | | | |
|---|---|---|---|---|---|---|
| S: Surfactant, P: Polyol, NA: Not Available **±: standard deviation | | | | | | |

### [Evaluations]

### (Stability)

The compositions according to Examples 1-12 and Comparative Examples 1-18 were maintained in an incubator at 45 °C for 1 month. The aspect of the compositions was visually observed and evaluated in accordance with the following criteria.
Good: No phase separation was observed
Poor: Phase separation was observed

The results are shown on the line labeled "Stability" in Tables 1-6.

### (Penetration Amount of Active Ingredient)

The penetration test experiments were performed in a Franz diffusion cell (Hanson) with a Strat-M membrane (Millipore, diameter of 15 mm). This equipment was composed of a donor part and a receptor part with the Strat-M membrane between the donor and receptor parts. The receptor part with a predetermined volume was filled with a receiving solution (0.25 wt% Tween 80/deionized water) maintained at a temperature of 32°C, which was continuously stirred with a small magnetic bar. Each of the compositions according to Examples 1-12 and Comparative Examples 1-18 was spread with a spatula on the membrane of the donor part with an amount of 30 mg/cm². After 5 hours, 200 µl of the receiving solution was withdrawn from the receptor part, while providing the same amount of a new receiving solution to the receptor part to maintain the same penetration conditions. The withdrawn receiving solution was analyzed by HPLC to determine the amount of the active ingredient in the receiving solution. The percentage of the active ingredient that passed through the membrane was calculated by dividing the detected amount of the active ingredient in the receiving solution by the amount of the active ingredient spread on the membrane.

The results are shown on the line labeled "Penetration Amount of Active Ingredient" in Tables 1-6.

The comparison of Example 1 and Comparative Examples 1-3 demonstrates that, in emulsions, the use of two polyglyceryl fatty acid esters enhanced the penetration of the active ingredient (ascorbyl glucoside) as compared to the use of a single or no polyglyceryl fatty acid ester.

Example 2 demonstrates that the use of two polyglyceryl fatty acid esters enhanced the penetration of the active ingredient (ascorbyl glucoside) even without an oil.

The comparison of Example 3 and Comparative Examples 4-6 demonstrates that, in emulsions, the use of two polyglyceryl fatty acid esters enhanced the penetration of the active ingredient (hydroxypropyl tetrahydropyrantriol) as compared to the use of a single or no polyglyceryl fatty acid ester.

Example 4 demonstrates that the use of two polyglyceryl fatty acid esters enhanced the penetration of the active ingredient (hydroxypropyl tetrahydropyrantriol) even without an oil.

The comparison of Example 5 and Comparative Examples 7-9 demonstrates that, in emulsions, the use of two polyglyceryl fatty acid esters enhanced the penetration of the active ingredient (3-O-ethyl ascorbic acid) as compared to the use of a single or no polyglyceryl fatty acid ester.

Example 6 demonstrates that the use of two polyglyceryl fatty acid esters enhanced the penetration of the active ingredient (3-O-ethyl ascorbic acid) even without an oil.

The comparison of Example 7 and Comparative Examples 10-12 demonstrates that, in emulsions, the use of two polyglyceryl fatty acid esters enhanced the penetration of the active ingredient (niacinamide) as compared to the use of a single or no polyglyceryl fatty acid ester.

Example 8 demonstrates that the use of two polyglyceryl fatty acid esters enhanced the penetration of the active ingredient (niacinamide) even without an oil.

The comparison of Example 9 and Comparative Examples 13-15 demonstrates that, in emulsions, the use of two polyglyceryl fatty acid esters enhanced the penetration of the active ingredient (salicylic acid) as compared to the use of a single or no polyglyceryl fatty acid ester.

Example 10 demonstrates that the use of two polyglyceryl fatty acid esters enhanced the penetration of the active ingredient (salicylic acid) even without an oil.

The comparison of Example 11 and Comparative Examples 16-18 demonstrates that, in emulsions, the use of two polyglyceryl fatty acid esters enhanced the penetration of the active ingredient (phenylethyl resorcinol) as compared to the use of a single or no polyglyceryl fatty acid ester.

Example 12 demonstrates that the use of two polyglyceryl fatty acid esters enhanced the penetration of the active ingredient (phenylethyl resorcinol) even without an oil.

## Claims

1. A composition comprising:
(b) at least one first polyglyceryl fatty acid ester having an HLB value of 13.0 or more;
(c) at least one second polyglyceryl fatty acid ester having an HLB value of 10.0 or less, preferably 9.5 or less, and more preferably 9.0 or less;
(d) at least one skin care active ingredient; and
(e) water,
wherein
the (d) skin care active ingredient is selected from the group consisting of niacinamide, ascorbyl glucoside, phenylethyl resorcinol, hydroxypropyl tetrahydropyrantriol, 3-O-ethyl ascorbic acid, salicylic acid, and a mixture thereof.

2. The composition according to Claim 1, wherein the average HLB of the (b) first polyglyceryl fatty acid ester(s) and the (c) second polyglyceryl fatty acid ester(s) in the composition ranges 12.5 to 13.0.

3. The composition according to Claim 1 or 2, wherein the (b) first polyglyceryl fatty acid ester comprises 2 to 4 glycerol units, preferably 3 or 4 glycerol units, and more preferably 4 glycerol units.

4. The composition according to any one of Claims 1 to 3, wherein the fatty acid moiety of the (b) first polyglyceryl fatty acid ester comprises 12 or fewer carbon atoms, preferably 11 or fewer carbon atoms, and more preferably 10 or fewer carbon atoms.

5. The composition according to any one of Claims 1 to 4, wherein the amount of the (b) first polyglyceryl fatty acid ester(s) in the composition ranges from 0.01% to 15% by weight, preferably from 0.05% to 10% by weight, and more preferably from 0.1% to 5% by weight, relative to the total weight of the composition.

6. The composition according to any one of Claims 1 to 5, wherein the (c) second polyglyceryl fatty acid ester comprises 2 to 4 glycerol units, preferably 2 or 3 glycerol units, and more preferably 2 glycerol units.

7. The composition according to any one of Claims 1 to 6, wherein the fatty acid moiety of the (c) second polyglyceryl fatty acid ester comprises 14 or more carbon atoms, preferably 16 or more carbon atoms, and more preferably 18 or more carbon atoms.

8. The composition according to any one of Claims 1 to 7, wherein the amount of the (c) second polyglyceryl fatty acid ester(s) in the composition ranges from 0.01% to 10% by weight, preferably from 0.05% to 5% by weight, and more preferably from 0.1% to 1% by weight, relative to the total weight of the composition.

9. The composition according to any one of Claims 1 to 8, wherein the weight ratio of the amount of the (b) first polyglyceryl fatty acid ester(s)/the (c) second polyglyceryl fatty acid ester(s)
is from 1 to 5, preferably from 1.5 to 4, and more preferably from 2 to 3.

10. The composition according to any one of Claims 1 to 9, wherein the composition comprises (a) at least one oil.

11. The composition according to Claim 10, wherein the amount of the (a) oil(s) in the composition ranges from 0.01% to 20% by weight, preferably from 0.05% to 15% by weight, and more preferably from 0.1% to 10% by weight, relative to the total weight of the composition.

12. The composition according to Claim 10 or 11, wherein the composition is in the form of a nano- or micro-emulsion.

13. A cosmetic process for treating the skin, comprising the step of applying the composition according to any one of Claims 1 to 12 to the skin.

## Patentansprüche

1. Zusammensetzung, umfassend:
(b) wenigstens einen ersten Polyglycerylfettsäureester, der einen HLB-Wert von 13,0 oder mehr aufweist;
(c) wenigstens einen zweiten Polyglycerylfettsäureester, der einen HLB-Wert von 10,0 oder weniger, vorzugsweise 9,5 oder weniger und besonders bevorzugt 9,0 oder weniger, aufweist;
(d) wenigstens einen Hautpflegewirkstoff; und
(e) Wasser,
wobei
der (d) Hautpflegewirkstoff aus der Gruppe bestehend aus Niacinamid, Ascorbylglucosid, Phenylethylresorcinol, Hydroxypropyltetrahydropyrantriol, 3-0-Ethylascorbinsäure, Salicylsäure und einem Gemisch davon ausgewählt ist.

2. Zusammensetzung nach Anspruch 1, wobei der durchschnittliche HLB des/der (b) ersten Polyglycerylfettsäureester(s) und des/der (c) zweiten Polyglycerylfettsäureester(s) in der Zusammensetzung im Bereich von 12,5 bis 13,0 liegt.

3. Zusammensetzung nach Anspruch 1 oder 2, wobei der (b) erste Polyglycerylfettsäureester 2 bis 4 Glycerineinheiten, vorzugsweise 3 oder 4 Glycerineinheiten und besonders bevorzugt 4 Glycerineinheiten, umfasst.

4. Zusammensetzung nach einem der Ansprüche 1 bis 3, wobei die Fettsäurekomponente des (b) ersten Polyglycerylfettsäureesters 12 oder weniger Kohlenstoffatome, vorzugsweise 11 oder weniger Kohlenstoffatome und besonders bevorzugt 10 oder weniger Kohlenstoffatome, umfasst.

5. Zusammensetzung nach einem der Ansprüche 1 bis 4, wobei die Menge des/der (b) ersten Polyglycerylfettsäureester(s) in der Zusammensetzung im Bereich von 0,01 Gew.-% bis 15 Gew.-%, vorzugsweise von 0,05 Gew.-% bis 10 Gew.-% und besonders bevorzugt von 0,1 Gew.-% bis 5 Gew.-%, bezogen auf das Gesamtgewicht der Zusammensetzung, liegt.

6. Zusammensetzung nach einem der Ansprüche 1 bis 5, wobei der (c) zweite Polyglycerylfettsäureester 2 bis 4 Glycerineinheiten, vorzugsweise 2 oder 3 Glycerineinheiten und besonders bevorzugt 2 Glycerineinheiten, umfasst.

7. Zusammensetzung nach einem der Ansprüche 1 bis 6, wobei die Fettsäurekomponente des (c) zweiten Polyglycerylfettsäureesters 14 oder mehr Kohlenstoffatome, vorzugsweise 16 oder mehr Kohlenstoffatome und besonders bevorzugt 18 oder mehr Kohlenstoffatome, umfasst.

8. Zusammensetzung nach einem der Ansprüche 1 bis 7, wobei die Menge des/der (c) zweiten Polyglycerylfettsäureester(s) in der Zusammensetzung im Bereich von 0,01 Gew.-% bis 10 Gew.-%, vorzugsweise von 0,05 Gew.-% bis 5 Gew.-% und besonders bevorzugt von 0,1 Gew.-% bis 1 Gew.-%, bezogen auf das Gesamtgewicht der Zusammensetzung, liegt.

9. Zusammensetzung nach einem der Ansprüche 1 bis 8, wobei das Gewichtsverhältnis der Menge des/der (b) ersten Polyglycerylfettsäureester(s) / des/der (c) zweiten Polyglycerylfettsäureester(s) 1 bis 5, vorzugsweise 1,5 bis 4 und besonders bevorzugt 2 bis 3, beträgt.

10. Zusammensetzung nach einem der Ansprüche 1 bis 9, wobei die Zusammensetzung (a) wenigstens ein Öl umfasst.

11. Zusammensetzung nach Anspruch 10, wobei die Menge des/der (a) Öls/Öle in der Zusammensetzung im Bereich von 0,01 Gew.-% bis 20 Gew.-%, vorzugsweise von 0,05 Gew.-% bis 15 Gew.-% und besonders bevorzugt von 0,1 Gew.-% bis 10 Gew.-%, bezogen auf das Gesamtgewicht der Zusammensetzung, liegt.

12. Zusammensetzung nach Anspruch 10 oder 11, wobei die Zusammensetzung in Form einer Nano- oder Mikroemulsion vorliegt.

13. Kosmetisches Verfahren zur Behandlung der Haut, das den Schritt des Auftragens der Zusammensetzung nach einem der Ansprüche 1 bis 12 auf die Haut umfasst.

## Revendications

1. Composition comprenant :
(b) au moins un premier ester d'acide gras polyglycérylique ayant une valeur HLB de 13,0 ou plus ;
(c) au moins un second ester d'acide gras polyglycérylique ayant une valeur HLB de 10,0 ou moins, de préférence de 9,5 ou moins, et plus préférablement de 9,0 ou moins ;
(d) au moins un principe actif de soin de la peau ; et
(e) de l'eau,
dans laquelle
le principe actif de soin de la peau (d) est choisi dans le groupe constitué par la niacinamide, le glucoside d'ascorbyle et le phényléthyl résorcinol, l'hydroxypropyl tétrahydropyrantriol, l'acide 3-O-éthylascorbique, l'acide salicylique, et un mélange de ceux-ci.

2. Composition selon la revendication 1, dans laquelle la valeur HLB moyenne du ou des premier(s) ester(s) d'acide gras polyglycérylique (b) et du ou des second(s) ester(s) d'acide gras polyglycérylique (c) dans la composition est comprise dans la plage allant de 12,5 à 13,0.

3. Composition selon la revendication 1 ou 2, dans laquelle le premier ester d'acide gras polyglycérylique (b) comprend 2 à 4 motifs glycérol, de préférence 3 ou 4 motifs glycérol, et plus préférablement 4 motifs glycérol.

4. Composition selon l'une quelconque des revendications 1 à 3, dans laquelle la fraction acide gras du premier ester d'acide gras polyglycérylique (b) comprend 12 atomes de carbone ou moins, de préférence 11 atomes de carbone ou moins, et plus préférablement 10 atomes de carbone ou moins.

5. Composition selon l'une quelconque des revendications 1 à 4, dans laquelle la quantité du ou des premier(s) ester(s) d'acide gras polyglycérylique (b) dans la composition est comprise dans la plage allant de 0,01 % à 15 % en poids, de préférence de 0,05 % à 10 % en poids, et plus préférablement de 0,1 % à 5 % en poids, par rapport au poids total de la composition.

6. Composition selon l'une quelconque des revendications 1 à 5, dans laquelle le second ester d'acide gras polyglycérylique (c) comprend 2 à 4 motifs glycérol, de préférence 2 ou 3 motifs glycérol, et plus préférablement 2 motifs glycérol.

7. Composition selon l'une quelconque des revendications 1 à 6, dans laquelle la fraction acide gras du second ester d'acide gras polyglycérylique (c) comprend 14 atomes de carbone ou plus, de préférence 16 atomes de carbone ou plus, et plus préférablement 18 atomes de carbone ou plus.

8. Composition selon l'une quelconque des revendications 1 à 7, dans laquelle la quantité du ou des second(s) ester(s) d'acide gras polyglycérylique (c) dans la composition est comprise dans la plage allant de 0,01 % à 10 % en poids, de préférence de 0,05 % à 5 % en poids, et plus préférablement de 0,1 % à 1 % en poids, par rapport au poids total de la composition.

9. Composition selon l'une quelconque des revendications 1 à 8, dans laquelle le rapport pondéral de la quantité du ou des premier(s) ester(s) d'acide gras polyglycéryliques (b)/du ou des second(s) ester(s) d'acide gras polyglycéryliques (c) est de 1 à 5, de préférence de 1,5 à 4, et plus préférablement de 2 à 3.

10. Composition selon l'une quelconque des revendications 1 à 9, dans laquelle la composition comprend (a) au moins une huile.

11. Composition selon la revendication 10, dans laquelle la quantité de la ou des huile(s) (a) dans la composition est comprise dans la plage allant de 0,01 % à 20 % en poids, de préférence de 0,05 % à 15 % en poids, et plus préférablement de 0,1 % à 10 % en poids, par rapport au poids total de la composition.

12. Composition selon la revendication 10 ou 11, dans laquelle la composition est sous la forme d'une nano- ou micro-émulsion.

13. Procédé cosmétique de traitement de la peau, comprenant l'étape d'application de la composition selon l'une quelconque des revendications 1 à 12 sur la peau.
